(19) Europäisches Patentamt

European Patent Office

Office européen des brevets

(11) **EP 4 707 321 A1**

(12) **EUROPEAN PATENT APPLICATION**

(43) Date of publication:
**11.03.2026 Bulletin 2026/11**

(21) Application number: **25197546.2**

(22) Date of filing: **22.08.2025**

(51) International Patent Classification (IPC):
**C08J 3/00** (2006.01)     **C08L 1/00** (2006.01)
**C08J 7/04** (2020.01)     **A41D 19/00** (2006.01)

(52) Cooperative Patent Classification (CPC):
(C-Sets available)
**C08J 7/0427; A41D 19/00; C09D 105/00;**
B29C 41/14; C08J 2307/02                (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **29.08.2024  MY PI2024005043**

(71) Applicant: **Grow Innovations Sdn Bhd
42100 Klang Selangor Darul Ehsan (MY)**

(72) Inventors:
• **Xin, Lim Chia
  42100 Klang (MY)**
• **Ci, Clara Wong Chia
  42100 Klang (MY)**
• **Chuan, Lai Fook
  42100 Klang (MY)**
• **Lui, Ng Kim
  42100 Klang (MY)**
• **Kum, Tan Sri Leong Hoy
  42100 Klang (MY)**

(74) Representative: **Zacco Denmark A/S
Arne Jacobsens Allé 15
2300 Copenhagen S (DK)**

(54) **BIOBASED HYDROGEL COATED ELASTOMERIC ARTICLE AND METHODS OF MAKING THE SAME**

(57)     The present invention relates to a bio-based hydrogel coating composition for elastomeric articles provided to overcome the issues of sweat accumulation, skin dryness, irritation, and difficulty in donning wet gloves, while offering an alternative to powdered elastomeric articles. The bio-based hydrogel coating is designed to enhance the wearability, comfort, and hygiene of said elastomeric article. This innovative formulation eliminates the need for powder-based coatings while providing a multipronged solution to overcome the issues of sweat accumulation, skin dryness, irritation, and difficulty in donning wet gloves.

**EP 4 707 321 A1**

(52)  Cooperative Patent Classification (CPC): (Cont.)

C-Sets
**C09D 105/00, C08K 5/053**

**Description**

**FIELD OF INVENTION**

The invention relates to coatings and elastomeric articles. Particularly to bio-based polymeric hydrogel coatings and elastomeric articles coated therewith, providing improvements to issues associated with prolonged glove wearing, including sweat accumulation due to occlusion, skin dryness caused by trans epidermal water loss, irritations from glove chemical residues, and donning difficulty during glove changing.

**BACKGROUND**

Elastomeric materials, such as those used for manufacturing elastomeric articles such as gloves, are widely employed in various industries and healthcare settings to provide barrier protection for the hands against a range of hazards, including chemicals, biological agents, and physical injuries. However, prolonged glove wearing can lead to several issues, including skin dryness, irritation, perspiration, and difficulty in donning wet gloves. To mitigate the potential health issues associated with powdered gloves, powderless gloves have been developed as a safer alternative.

Several patents such as US20060062815A1, US5614202A, US20070053958A1, US6274154B1, US6423328B2 and US6630152B2 disclose gloves incorporating moisturizing or soothing agents to alleviate skin issues caused by glove wearing. Additionally, gloves with specific coatings or materials have been designed to facilitate easier donning, particularly in wet conditions such as disclosed in patents US20080034467A1, US8313833B2, WO2006071308, US20220175068A1, US20050132466A1 and US20060141186A1. There have also been developments to eliminate glove-related allergens by developing accelerator-free gloves as disclosed in CA3042804C and US10023718B2.

The challenges associated with glove use are multifaceted. Prolonged glove wear can lead to moisture accumulation which contributes to skin irritation and discomfort. Powdered gloves, once widely used, are now recognized as a health hazard due to the potential for inhalation and skin irritation. Additionally, the need for moisturizing agents to soothe and protect the skin during extended glove use is crucial. To prevent chemical exposure and skin reactions, gloves should also provide an effective barrier against chemical allergens. Finally, ease of donning is essential to minimize the risk of contamination and enhance user experience. In particular, none of the existing powderless elastomeric articles effectively addresses the above issues. Neither can any of the existing powderless elastomeric articles provide an elastomeric article which is able to address multiple issues such as overcoming sweat accumulation while simultaneously providing a moisturizing effect, a barrier against chemical allergens and provide ease of donning to a user.

It is an aim of the present invention therefore to provide an elastomeric article, such as a glove, coated with a bio-based polymeric (biopolymeric) hydrogel that addresses the issues associated with prolonged glove wear. Specifically, the invention seeks to overcome the issues of sweat accumulation, skin dryness, irritation, and difficulty in donning wet gloves, while offering an alternative to powdered elastomeric articles. The bio-based hydrogel coating is designed to enhance the wearability, comfort, and hygiene of said elastomeric article.

**SUMMARY OF INVENTION**

It is an embodiment of the present invention to provide a biopolymeric hydrogel composition for use as a coating for an elastomeric article comprising: a biopolymeric hydrogel formed from polysaccharides extracted from mushrooms; glycerol; preserving agents; and thickening agents wherein the polysaccharides are extracted from any one or in combination of *Tremella fuciformis, Lentinula edodes ,Ganoderma lucidim or Inonotus Obliquus*.

It is an embodiment of the present invention to provide a biopolymeric hydrogel composition for use as a coating wherein the composition exhibits improved moisture absorption properties, the composition exhibits skin hydrating properties, the composition exhibits physical barrier properties, or the composition provides a user with ease of donning.

It is an embodiment of the present invention to provide a method for manufacturing an elastomeric article with a biopolymeric hydrogel composition coating, comprising the steps of:

- preparing and cleaning a former;
- applying a coagulant to the former;
- dipping the former into an aqueous polymer latex to form a body of the article;
- pre-leaching and subsequent cooling of the formed article;
- chlorinating and neutralizing the article;
- immersing the article into a coating composition for coating an outer surface of the article;
- drying the coated article; and
- removing the article from the former, with the outer surface becoming an inner surface with a coating via inverted stripping or equivalent means;

wherein the characterized in that the coating composition is a biopolymeric hydrogel coating according to an embodiment above.

It is an embodiment of the present invention to provide an elastomeric article with a biopolymeric hydrogel composition comprising: an outer surface and inner surface formed from an elastomeric material; wherein the inner surface is applied with a biopolymeric hydrogel composition according to any one of the above embodiments.

It is an embodiment of the present invention to provide an elastomeric article with a biopolymeric hydrogel composition comprising elastomeric material wherein the elastomeric article is formed from elastomeric material comprised of biologically based monomers selected but not limited to bio-butadiene monomers, bio-acrylonitrile monomers, or bio-methacrylic acid monomers.

## BRIEF DESCRIPTION OF THE DRAWINGS

Figure 1 illustrates an exemplified overview of the synthesis methodology of the biopolymeric hydrogel composition for coating an elastomeric article.

Figure 2 illustrates conventional means of forming an elastomeric glove with a coating.

Figure 3 illustrates results of user perception study comparing the non-coated glove against the biopolymeric hydrogel coated glove.

## DETAILED DESCRIPTION OF THE INVENTION

It is to be understood that the embodiments disclosed herein are illustrative of the principles of the claims. Other modifications that may be employed are within the scope of the claims. Thus, by way of example, but not of limitation, alternative embodiments may be utilized in accordance with the disclosure herein.

Accordingly, the claims are not limited to embodiments precisely as shown and described. Throughout this specification and the claims, which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this disclosure relates.

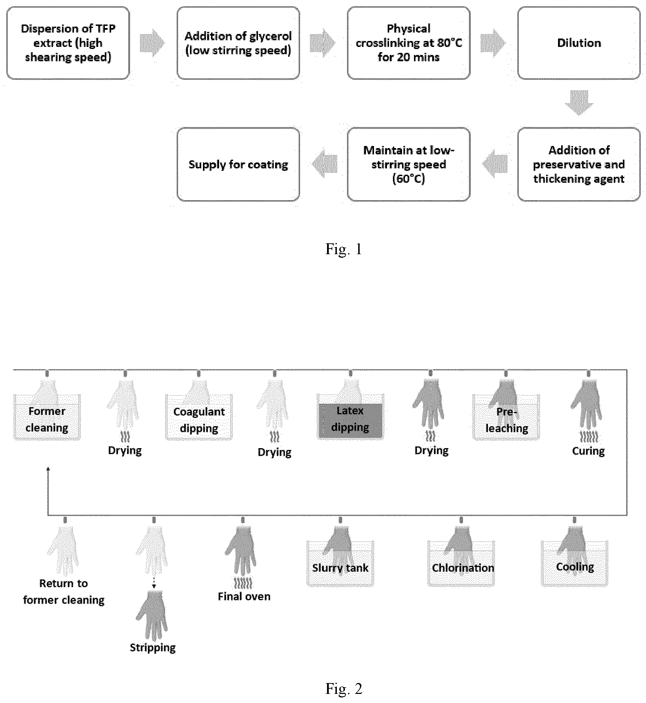

Figure 1 illustrates an exemplified overview of synthesis methodology of a biopolymeric hydrogel derived from *Tremella fuciformis polysaccharide* (TFP) composition used for coating an elastomeric article.

It is an embodiment of the present invention to provide a composition for use as a coating for an elastomeric article comprising a polymer hydrogel formed using mushroom polysaccharides extracts extracted from mushrooms; glycerol; preserving agents; and thickening agents.

The polymer hydrogel is formed using a synthesis process illustrated in figure 1. The polymer hydrogel is formed beginning with a formation of a dispersion containing polysaccharides obtained from but not limited to mushrooms. Glycerol is incorporated to the dispersion along with preserving and thickening agents.

In a further embodiment of the present invention, it is provided a composition for providing a composition for use as a coating for an elastomeric article comprising a polymer hydrogel according to the following steps:

(a) 5 % by mass of TFP extracts relative to the mass of water from a source of polysaccharides such as but not limited to *Tremella fuciformis* is combined with water to form a dispersion using a high-speed mixer which provides high shearing conditions preferably at around 8000 rpm. The shearing process is continued until the dispersion mixture becomes a homogeneous mixture;

(b) 2 % by mass of Glycerol relative to the mass of TFP is then incorporated to the dispersion and stirring is continued at a low speed for preferably about 20 minutes at a temperature of about 80 °C. This step allows for significant crosslinking of the polysaccharides. The physical crosslinking is facilitated by the addition of glycerol which interacts with the carboxyl and hydroxyl groups within the polysaccharide chain via mechanisms of hydrogen bonding and disruption of hydrophobic interactions;

(c) The low speed stirring process is maintained at preferably 80 rpm throughout the crosslinking process.

(d) Additional thickeners and preservatives are added to the starch dispersion slurry to enhance viscosity and prevent microbial growth during storage.

It is to be noted that the use of TFP extracts is this disclosure is interchangeable with any alternative mushroom source which acts as a polysaccharide source.

In the processing of the composition for preparation of the polymer hydrogel coating, the formation of the polysaccharide dispersion comprises a polysaccharide source, glycerol, thickeners, and preservatives. The polysaccharide sources are selected from but not limited to *Snow mushroom (Tremella fuciformis), Shiitake mushroom (Lentinula edodes), Chaga mushroom (Inonotus obliquus), Reishi mushroom (Ganoderma lucidum) or any other similar mushroom species. Tremella fuciformis* for example being a natural and sustainable mushroom, offers a compelling alternative to traditional synthetic materials for glove coatings. Its biodegradability and reduced chemical footprint contribute significantly to environmental sustainability, making it a preferred choice for eco-conscious consumers and businesses. Its natural properties and generally safe nature minimize potential health hazards associated with some synthetic materials, making it a safer choice for consumers.

Glycerol in the present invention is mainly used as a crosslinking agent. However, it is to be understood that other crosslinking agents such as propylene glycol, polyethylene glycol, sorbitol and others can provide an equivalent crosslinking effect. Thickening agents (thickeners) are selected but not limited to gums like guar gum, xanthan gum, locust bean gum, and Arabic gum; cellulose derivatives such as methylcellulose, carboxymethyl cellulose (CMC), hydroxyethyl cellulose (HEC), and microcrystalline cellulose and synthetic polymers like polyacrylamide and polyvinyl alcohol (PVA). Preservatives are selected but not limited to phenoxyethanol, chlorphenesin, potassium sorbate and sodium benzoate.

It is an object of the present invention to prepare a composition for use as a coating for an elastomeric article that exhibits a high moisture absorption when compared to existing coatings.

The polymer hydrogel coating is formed from the TFP extract which readily forms the homogeneous hydrogel dispersion. Hydrogels absorb moisture through both water-binding and retention mechanisms, starting at the surface of the hydrogels. The absorption process begins when water molecules interact with the hydrophilic functional groups, such as hydroxyl (-OH) groups in the TFP chain, located on the hydrogel's surface. These hydroxyl groups, abundant in macromolecular polymer chains, have a strong affinity for water and form hydrogen bonds with surrounding water molecules. As water interacts with the surface, it diffuses into the hydrogel's complex which is comprised of interconnected three-dimensional network structures formed by a crosslinking process. This crosslinked network creates a stable matrix of interconnected polymer chains that form spaces or pores which are able to physically trap water molecules within its structure. This network structure (established through crosslinking) is crucial for maintaining the hydrogel's integrity and preventing the polymer chains from dissolving or separating once moisture has been absorbed.

It is an object of the present invention to provide a composition for use as a coating for an elastomeric article which is able to provide a user a moisturizing effect and thus providing skin hydration while the elastomeric article is in use.

Hydrogel are polymeric materials with a high water-holding capacity, also offer an effective way to moisturize hands when incorporated into gloves. Upon interacting with moisture, TFP components of a smaller molecular weight diffuse from the hydrogel matrix and engage with the skin's surface. Due to their small size, these TFP molecules penetrate the stratum corneum (SC) and reach the deeper epidermal layers. At this point, the TFP molecules in the epidermal layers form a hydrophilic matrix that binds and retains both internal and sweat-derived moisture. This process establishes a moisture-retentive environment within the epidermis, enhancing hydration and improving overall skin moisture balance. The moisturizing effect can be further adjusted by varying the TFP molecular weight, coating thickness, and hydrogel composition.

It is an object of the present invention to provide a composition for use as a coating for an elastomeric article which is able to provide a physical barrier layer which protects the skin from direct contact with chemical residues or allergens present in the elastomeric article.

Elastomeric articles such as gloves, while essential for protection in various industries, can inadvertently harbor allergens and chemicals that may pose risks to the wearer. These contaminants can originate from the manufacturing process, environmental exposure, or even the specific use of gloves. For example, latex gloves often contain proteins that can trigger allergic reactions in sensitive individuals, while residual accelerators in the gloves can trigger type-IV hypersensitivity in some people. To mitigate these risks and safeguard the wearer's health, a chemical barrier coating is crucial. Such a barrier can effectively prevent the transfer of allergens and chemicals from the glove surface to the skin, thereby reducing the likelihood of adverse reactions and ensuring wearer safety. The biopolymeric hydrogel composition which forms the coating for an elastomeric article such as a glove provides a barrier to effectively shield the skin from direct contact with harmful chemical residues or allergens that may be present within the elastomeric material itself or that may adhere to the surface during use. This coating forms an interposing physical barrier between the skin and potential irritants which aims to significantly reduce the risk of skin reactions, allergies, and other adverse health effects associated with glove use.

It is an object of the present invention to provide a composition for use as a coating for an elastomeric article which is able to provide a user with ease of donning.

The process of putting on an elastomeric article such as a glove can be referred to as 'donning' or 'donnability' When considering the donnability of an elastomeric article such as a glove, one of the means of determining donnability is by studying the friction coefficient via friction testing. Friction coefficient is a dimensionless number that quantifies the amount of friction between two surfaces in contact. It is a measure of how resistant these surfaces are to sliding or slipping past each other. A lower frictional coefficient would indicate a smoother surface between the two surfaces, hence meaning easier donning. The presence of a TFP hydrogel coating results in an overall reduction of the friction coefficient which expedites the donning process for both dry and damp skin conditions. Specifically in the use of gloves, it would expedite donning process under both dry and damp hand conditions due to the slippery hydrogel texture and the exceptional ability of the hydrogel coating to absorb water which readily absorbs moisture from the surface of a user's hand.

It is therefore an embodiment of the present invention to provide an elastomeric article incorporating a biopolymeric hydrogel composition for use as a coating on said elastomeric article according to the following steps (as shown in figure 2):

(a) A former is prepared and cleaned using acid and alkali wash to remove dirt and residue. A first coat of coagulant typically comprising but not limited to calcium nitrate or carbonate as a latex coagulant and stearate as an antitack agentis applied to the formers to ensure proper glove formation and smooth stripping from said former. Optionally, wetting agent including Teric 320 can be added to ensure proper former surface wetting for homogenous coagulant coating.

(b) The composition is then dipped into aqueous polymer latex which forms the body of the glove. Latex dipping can involve a single or multiple dipping process to achieve a desired thickness.

(c) The elastic article formed from the process undergoes pre-leaching to remove excess proteins and impurities, then curing process in an oven to allow warm air to circulate to dry remaining moisture via evaporation and facilitate glove crosslinking process. After crosslinking, the glove exhibits increased durability, resistance to tearing and stretching, improved chemical and heat resistance, and enhanced structural stability.

(d) Once the curing process is completed, the final article is subjected to cooling process, and then undergoes chlorinization by dipping the elastomeric article into an aqueous chlorinated solution at 800 - 1200 ppm to improve surface properties. Proper chlorination makes the gloves less tacky and smoother for easy donning.

(e) After chlorination, the glove is subjected to neutralization by washing the gloves with a base such as sodium hydroxide or ammonia to remove residual chlorine and adjust the surface pH and post-leaching process to remove remaining impurities.

(f) The elastomeric article is then immersed into the TFP dispersion slurry formed by the process of figure 1 (as described in the description of the patent application above) whereby the outer surface of the glove is coated with the TFP dispersion slurry.

(g) The elastomeric article then undergoes further drying to allow removal of water which forms a solid coating on the outer surface of the elastomeric article.

(h) The coated elastomeric article is then removed from the former via inversion stripping process, whereby the outer surface (while on the former) is inverted to become the inner surface of the elastomeric article as depicted by figure 1. With this production methodology, the final glove exhibits enhanced moisture absorption, moisturizing properties, good donning properties, physical barrier protection and soothing effect facilitated by the biopolymeric hydrogel coating.

It is an object of the present invention to prepare an elastomeric article such as a glove via the method above which comprises of an outer surface and an inner surface. The biopolymeric hydrogel is applied on the outer surface which is then inverted to become the inner surface. As such, the final article produced would have an outer surface and an inner surface coated with the biopolymeric hydrogel coating.

It is an object of the present invention to prepare an elastomeric material which can be considered to be thin and powder free. The use of the biopolymeric hydrogel coating as disclosed herein can create an ultra-thin film on the elastomeric materials (e.g. gloves), resulting in a powderless design. This thin layer of coating absorbs moisture from the skin, reducing the need for traditional powder coatings that can cause allergic reactions or leave residue. The hydrogel coating also provides enhanced moisture absorption, moisturizing properties, good donning properties, physical barrier protection and soothing effect, making them ideal for prolonged wearing in various applications, especially in healthcare and industrial settings. When assessed according to the ASTM D6124 standard, a mass of less than 2 mg/glove is necessary to be regarded as powder-free gloves. The biopolymeric hydrogel coating when incorporated to the gloves would produce a glove with mass of less than 2mg thus fulfilling the ASTM standards.

In terms of thickness of the elastomeric article, it is preferred for the glove to be less than 0.2 mm thin, preferably 0.05 mm to 0.2 mm in thickness. Gloves which are less than 0.2 mm thick are considered to be in the category of thin gloves. Gloves of this thickness are designed to provide enhanced tactile sensitivity, dexterity, and comfort for applications demanding precision and fine motor skills. These gloves are particularly suitable for medical procedures requiring delicate manipulations, such as microsurgery, and industrial tasks involving small or delicate components. The reduced bulk and improved comfort of ultra-thin gloves contribute to enhanced hand-eye coordination, reduced fatigue, and overall improved performance in tasks requiring a high degree of tactile sensitivity and precision. It would therefore be an object of the present invention to prepare an elastomeric article such as a glove which is considered thin gauge gloves which would be less than 0.2 mm in thickness.

It is an object of the present invention to prepare an elastomeric material which is fully biologically based and sustainable. The novel elastomeric article if formed from an elastomeric material incorporating one or more bio-based monomers selected from but not limited to bio-butadiene monomers, bio-acrylonitrile monomers, or bio-methacrylic acid monomers. These bio-based monomers are derived from renewable and sustainable biological sources, significantly reducing the carbon footprint associated with the production of the elastomeric article compared to conventional articles utilizing petroleum-derived monomers. The incorporation of these bio-based monomers promotes the development of a sustainable elastomeric material used to manufacture elastomeric articles while potentially enhancing performance characteristics, including durability, flexibility, chemical resistance, adhesion, crosslinking efficiency, and other desired properties.

Latex and bio latex are both natural rubber materials, but they differ significantly in their source and allergenicity. Latex is derived from rubber trees. Bio latex, on the other hand, is derived from plant-based sources other than rubber trees. Bio latex production may have a lower environmental impact compared to traditional latex production and offers a more sustainable alternative to traditional latex derived from rubber trees. The production of bio latex often involves less intensive agricultural practices and may have a lower overall environmental impact compared to latex production. Additionally, bio latex contributes to a reduced carbon footprint due to its reliance on renewable plant-based resources. Therefore, the use of bio latex in various applications can contribute to a more sustainable and environmentally responsible approach. Bio latex is a natural rubber material composed of a long chain of repeating monomer units (such as bio-butadiene monomers, bio-acrylonitrile monomers, or bio-methacrylic acid monomers).

It is therefore a further embodiment of the present invention to provide an elastomeric article incorporating a biopolymeric hydrogel composition for use as a coating on said elastomeric article according to the following steps (as shown in figure 2) wherein the conventional polymer latex is replaced with polymer latex made from fully biologically based (bio based) monomers selected from but not limited to bio-butadiene monomers, bio-acrylonitrile monomers, or bio-methacrylic acid monomers:

(a) A former is prepared and cleaned using acid and alkali wash to remove dirt and residue. A first coat of coagulant typically comprising but not limited to calcium nitrate or carbonate as a bio based latex coagulant and stearate as an antitack agent is applied to the formers to ensure proper glove formation and smooth stripping from said former. Optionally, wetting agent including Teric 320 can be added to ensure proper former surface wetting for homogenous coagulant coating.

(b) The composition is then dipped into aqueous bio-based polymer latex which forms the body of the glove. Latex dipping can involve a single or multiple dipping process to achieve a desired thickness.

(c) The elastic article formed from the process undergoes pre-leaching to remove excess proteins and impurities, then curing process in an oven to allow warm air to circulate to dry remaining moisture via evaporation and facilitate glove crosslinking process. After crosslinking, the glove exhibits increased durability, resistance to tearing and stretching, improved chemical and heat resistance, and enhanced structural stability.

(d) Once the curing process is completed, the final article is subjected to cooling process, and then undergoes chlorinization by dipping the elastomeric article into an aqueous chlorinated solution at 800 - 1200 ppm to improve surface properties. Proper chlorination makes the gloves less tacky and smoother for easy donning.

(e) After chlorination, the glove is subjected to neutralization by washing the gloves with a base such as sodium hydroxide or ammonia to remove residual chlorine and adjust the surface pH and post-leaching process to remove remaining impurities.

(f) The elastomeric article is then immersed into the TFP dispersion slurry formed by the process of figure 1 (as described in the description of the patent application above) whereby the outer surface of the glove is coated with the TFP dispersion slurry

(g) The elastomeric article then undergoes further drying to allow removal of water which forms a solid coating on the outer surface of the elastomeric article.

(h) The coated elastomeric article is then removed from the former via inversion stripping process, whereby the outer surface (while on the former) is inverted to become the inner surface of the elastomeric article as depicted by figure 1. With this production methodology, the final glove exhibits enhanced moisture absorption, moisturizing properties, good donning properties, physical barrier protection and soothing effect facilitated by the biopolymeric hydrogel coating.

**METHODOLOGY**

In the comparative example below, the following equipment was used in the processing of the composition in lab-scale.

1. Silverson mixer (L5M-A, Silverson, USA)
2. Overhead stirrer (EUROSTAR 60 digital, Ika®-Werke GmbH & Co. KG, Germany)
3. Water bath heating system

Materials used in the example(s)

The materials and methods described herein are provided as examples and are not intended to be limiting. It is to be understood that other materials and methods may be used to practice the invention, and the scope of the invention is defined only by the appended claims.

Mushroom polysaccharide extract including Snow mushroom *(Tremella fuciformis)*, Shiitake *(Lentinula edodes)*, Chaga *(Inonotus obliquus)*, Reishi *(Ganoderma lucidum)*, etc. can be used as the main component for synthesis of the renewable polymer hydrogel in this invention. TFP extract was commercially acquired from Shandong Focusfreda Biotech Co., Ltd (Treme-HA®, China) as the exemplary candidate for the hydrogel dispersion. Glycerol (USP 99.7%) was procured from Stallion Chemical (M) Sdn. Bhd. (Malaysia). Preservative and thickening agent were supplied by Shanghai Cosroma Biotech Co., Ltd (China) and Xi 'an Best Bio-Tech Co., LTD. (China), respectively.

Renewable NBR latex (bio latex) used for producing gloves is a bio-based alternative to traditional petrochemically-derived NBR latex. It is produced from Bio-Butadiene, Bio-Acrylonitrile and Bio-Methacrylic acid as renewable natural photosynthesis or artificial photosynthesis route biomass feedstock such as glucose or other sugars, starch-based materials, and greenhouse gas (GHG) $CO_2$. The chemistry synthesis and physical characteristics of renewable NBR latex (bio latex) are identical to those of NBR latex generated from petrochemicals.

Synthesis methodology

The overview of the synthesis process of the biopolymeric hydrogel coating as illustrated in figure 1 and disclosed above is as follows:

The formation of a polymer hydrogel begins with the dispersion of 5 % (w/w) renewable *Tremella fuciformis* polysaccharide (TFP) extract in water at 8000 rpm under high shearing Silverson mixer until homogeneity is achieved. Glycerol was incorporated at 2 % (w/w) relative to TFP mass and the dispersion was allowed to stir at lower speed with an overhead stirrer for 30 mins. The TFP dispersion was subjected to heat at 80 °C for 20 minutes to achieve sufficient physical crosslinking for the formation of hydrogel dispersion. Physical crosslinking of TFP chains was facilitated by glycerol as the crosslinking agent whereby it interacts with the carboxyl and hydroxyl groups within the polysaccharide chain. Stirring speed was maintained at 80 rpm with a flat-blade propeller (10 cm X 10 cm) during the crosslinking process. The hydrogel dispersion was then diluted to 2 % (w/w) and maintained at 60 °C via continuous stirring at 80 rpm. Preservative and thickening agent were added at the final stage at 0.02 % (w/w) and 0.01 % (w/w), respectively.

The TFP hydrogel was then coated onto a rubber glove via the dip coating technique before the final oven stage. Rubber elastomeric glove produced with the bio-based latex following the conventional glove manufacturing practice (as shown in figure 2 and disclosed above) was immersed in the hydrogel coating solution for a predetermined dwell time. The layer of hydrogel coating was dried in the final oven before being inverted via the stripping process, such that the hydrogel-coated surface facing outward previously becomes the inner surface of the glove, namely the donning side.

*In vitro* moisture absorption capacity of glove

The absorption capacity of the hydrogel coating was evaluated via an *in vitro* technique modified from the ISO 9073-6:2000 standard used to assess nonwoven absorption. 600 mg of 0.5 % (w/v) sodium chloride solution as artificial sweat was spread evenly on the internal surface of the pre-weighed glove. The glove was then left under a controlled environment for 20 minutes to simulate wearing conditions in clinical scenarios. The internal coatings were then exposed to the external environment under a fume hood for 2 minutes to remove any excess liquid. The gloves were then weighed, and the absorption capacity was determined based on the results of four replications.

The absorption capacity is then calculated based on the following formula:

$$Absorption\ capacity\ (mg) = Final\ weight\ of\ glove - Initial\ weight\ of\ glove$$

Assessment of powder content in glove

The powder content of the TFP hydrogel-coated gloves was assessed according to the ASTM D6124 standard whereby fulfillment of less than 2 mg/glove is necessary to be regarded as powder-free gloves.

Determination of Coefficient of friction (COF, $\mu$)

The coefficient of friction ($\mu$) of the hydrogel-coated rubber substrate was measured using a COF tester (MH2-500N, IMADA Co., LTD, Japan) coupled with a digital force gauge (ZTS-5N, IMADA Co., LTD, Japan). The method of analysis was adopted from ASTM D1894 standard specifications under dry and damp conditions. The testing samples were cut into a size of 250 mm x 130 mm and mounted onto the plane with the donning side facing upwards. A 200 g sled was allowed to slide against the testing surface at a speed of 150 mm/min in cycle mode. The average static $\mu$ values were calculated from triplicates for each sample set, as computed by the Force Recorder Standard software.

For testing under damp conditions, the sled surface was wet with approximately 200 mg of deionized water. The water was evenly distributed by swiping back and forth five times horizontally and vertically across the sled surface. The subsequent procedure followed as described above.

Table 1 shows the comparison of moisture absorption capacity, powder content and surface friction coefficient between the biopolymeric hydrogel coated gloves and non-coated gloves.

Table 1 - Comparison of moisture absorption capacity, powder content and surface friction coefficient between the biopolymeric hydrogel coated gloves and non-coated gloves.

| Parameters | Negative control (non-coated glove) | TFP hydrogel-coated glove |
|---|---|---|
| Powder content (mg/glove) | 0.10 | 0.16 |
| Percentage increase in absorption (%) | - | 146 |
| Percentage reduced in friction coefficient under dry condition (%) | - | 30.0 |
| Percentage reduced in friction coefficient under damp condition (%) | - | 41.4 |

The example above displays the gel-forming properties of TFP that readily dissolve in water to form homogenous hydrogel dispersion. TFP hydrogel is formed via physical crosslinking with the aid of glycerol as a crosslinker between the chains of TFPs, resulting in a continuous network of film upon drying. The powder content of 0.16 mg/glove detected for the TFP hydrogel-coated glove confirmed its identity as a powderless coating (Table 1). A proper microstructure of the hydrogel matrix is necessary for an excellent water-capturing and retention ability. As shown in table 1, TFP hydrogel exhibited significantly higher (p = 0.0005) *in vitro* absorption capacity (105.5 mg) relative to negative control (40.2 mg), 146 % improvement in absorption. The exquisite water-holding capacity of the hydrogel is attributed to the abundant hydroxyl (OH) groups within the macromolecular biopolymeric network chain that can capture and entrap the surrounding water molecules via hydrogen bonding.

Friction tests were conducted to study the friction coefficient between two surfaces, namely the sled and the elastomeric glove. The donnability of gloves strongly correlates to the friction coefficient, with a lower frictional force indicates a smoother surface and hence easier donning. As presented in Table 1, the presence of TFP hydrogel coating resulted in an overall reduction of 30 % in the average static coefficient of friction ($\mu$) under dry conditions (TFP hydrogel: 0.40±0.01; Negative control: 0.57±0.03). Whereas under moist conditions (TFP hydrogel: 0.75±0.05; Negative control:

1.28±0.07), approximately 41 % reduction in static $\mu$ is recorded. This suggests that TFP hydrogel coating would expedite the donning process under both dry and damp hand conditions due to the slippery hydrogel texture and the exceptional water-imbibing ability that readily removes moisture from the hand surface.

## User perception study

A user perception study was conducted to evaluate several parameters including donning efficacy under damp and dry hand conditions, sweat absorption efficacy, moisturizing effect, and lastly barrier protection and soothing effect against potential glove allergens. All glove samples were conditioned at room temperature for 24 hrs. Before the study, subjects were requested to wash their hands thoroughly before wearing gloves. Treated (TFP hydrogel coated) and non-treated (non-coated/negative control) gloves were provided to the subjects in a blinded and random manner. The position of gloves for each subject was documented and was repeated on the next day with gloves provided in a reversed position. The participants were allowed to continue with their routine during the test period of either 10 minutes or 60 minutes. The 10-minute test was used to assess features including damp donning, sweat absorption, and moisturizing effect whereas the 60-minute test evaluates dry donning as well as the barrier protection and soothing feature of the coating. To assess the damp donning property, participants were subjected to sprays of a total 600 mg artificial sweat (0.5 % w/w NaCl). Two sprays were given each on the palmar and dorsal surface followed by two times of rubbing to allow even distribution on the palm. On the contrary, gloves were directly donned with dry hands for the evaluation of the dry donning property. User feedback was collected on a questionnaire basis on a five-point Likert scale of 1 to 5 (strongly disagree to strongly agree).

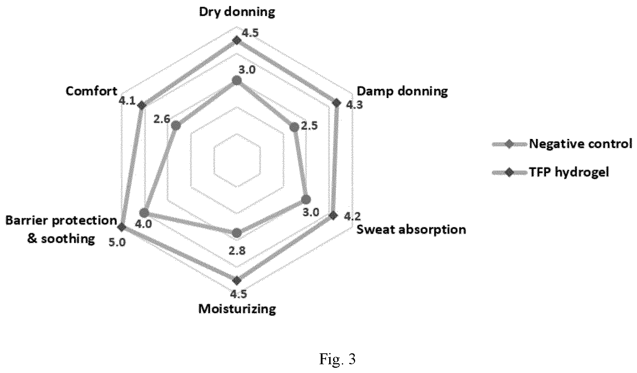

Several features were evaluated via the user perception study including glove donnability under dry and damp hand conditions, sweat absorption, moisturizing effect, barrier protection and soothing against glove residual chemicals. The results were summarized in figure 3 based on the average of Likert scale ratings obtained for each feature assessed. Donnability of the glove was assessed based on the ease of donning, the level of resistance, and the level of adjustment required during donning. Subjects were then asked to rate their level of agreement towards the statement of easy donning for each side of the glove. All subjects experienced faster and more straightforward dry donning with less resistance and adjustment needed for the TFP hydrogel (TFP hydrogel: 4.5; negative control: 3.0). The difference in perceived donnability was more apparent when donning was performed with damp hands where approximately 70 % of differences were determined based on the average score rated (TFP hydrogel: 4.3; negative control: 2.5). The results were in accordance with the significant reduction of friction coefficient relative to the negative control (Table 1), hence indicating an improved donnability of TFP hydrogel-coated gloves.

The amount of sweat felt on hand surfaces after doffing was used as the assessment criterion for sweat absorption efficacy. 60 % of the subjects had positive responses (TFP hydrogel: 4.2; Negative control: 3.0) towards the sweat absorption of TFP hydrogel after 10-minute wear albeit with a 146 % increase in absorption measured experimentally (Table 1). This may be due to the absorption at the milligram (mg) level which is a minuscule amount to be felt by every subject. On the contrary, moisturizing effect was evaluated based on both visual and tactile judgement after glove removal. A majority (80 %) of the subjects felt the moisturizing effect within a 10-minute wear of TFP hydrogel where smooth and silky was part of the feedback provided by the positive respondents. The moisturizing effect was attributed to the ability of TFP in penetrating the SC to the center of the epidermis for moisture retention and function as a hydration barrier on the SC to minimize trans epidermal water evaporation.

Furthermore, mild skin irritation, particularly itching and dryness was encountered by most of the subjects when negative control (4.0) was worn for a prolonged period (60 minutes). It is noteworthy that the absence of skin irritation was reported for TFP hydrogel (5.0) by all the respondents. This implies that the TFP hydrogel acts as a layer of physical barrier that protects the skin from direct contact with chemical residues or allergens in the glove in addition to its soothing ability. In sum, a higher grading score was rated for TFP hydrogel (4.1) for overall comfort compared to the negative control (2.6). As a whole, perceptions of TFP hydrogel-coated gloves were better than the negative control. 80% of the subjects agreed that TFP hydrogel provides superior wearing experience and expressed a preference for TFP hydrogel-coated gloves. The enhanced comfort is attributed to the collective advantages offered by TFP hydrogel including easier dry and damp donning, sweat absorption, moisturizing, barrier protection, and soothing.

## Statistical analysis

All measurements were performed in multiple replications as specified in respective sections with results expressed as the mean ± standard error (SE). GraphPad Prism 8.0.2 (GraphPad, CA, USA) was used for graphical representation of data and for determination of the statistical significance ($p < 0.05$) between mean values for which the student t-test was employed.

**Claims**

1. A biopolymeric hydrogel composition for coating for an elastomeric article comprising a biopolymeric hydrogel formed from polysaccharides extracted from mushrooms.

2. A biopolymeric hydrogel composition for use as a coating for an elastomeric article comprising the biopolymeric hydrogel according to claim 1, glycerol, preservatives, and thickening agents.

3. The biopolymeric hydrogel composition of any one of claims 1 or 2 wherein the composition exhibits improved moisture absorption properties.

4. The biopolymeric hydrogel composition of any one of claims 1 to 3 wherein the composition exhibits skin hydrating properties.

5. The biopolymeric hydrogel composition of any one of claims 1 to 4 wherein the composition exhibits physical barrier properties which provides a user with barrier protection and soothing properties.

6. The biopolymeric hydrogel composition of any one of claims 1 to 5 wherein the composition facilitates easy donning.

7. The biopolymeric hydrogel composition of any one of claims 1 to 6, wherein the polysaccharides are extracted from any one or in combination of *Tremella fuciformis, Lentinula edodes, Ganoderma lucidim or Inonotus Obliquus.*

8. A method for manufacturing an elastomeric article with a biopolymeric hydrogel composition coating, comprising the steps of:

   - preparing and cleaning a former;
   - applying a coagulant to the former;
   - dipping the former into an aqueous polymer latex to form a body of the article;
   - pre-leaching and subsequent cooling of the formed article;
   - chlorinating and neutralizing the article;
   - immersing the article into a coating composition for coating an outer surface of the article;
   - drying the coated article; and
   - removing the article from the former, with the outer surface becoming an inner surface with a coating via inverted stripping or equivalent means;

   **characterized in that** the coating composition is a biopolymeric hydrogel coating according to any one of claims 1-7

9. An elastomeric article with a biopolymeric hydrogel composition comprising:

   an outer surface and
   inner surface formed from an elastomeric material;
   **characterized in that** the inner surface is applied with a biopolymeric hydrogel composition according to any one of claims 1-7.

10. The biopolymeric hydrogel composition of claims 1 to 7, method of claim 8 or elastomeric article of claim 9 wherein the elastomeric article is formed from an elastomeric material formed from monomers of biologically based monomers.

11. The biopolymeric hydrogel composition of any one of claims 1 to 6, method of claim 8 or elastomeric article of claim 9 wherein the elastomeric article is formed from an elastomeric material formed from monomers selected from bio-butadiene monomers, bio-acrylonitrile monomers, or bio-methacrylic acid monomers.

12. The biopolymeric hydrogel composition of any one of claims 1 to 7, method of claim 8 or elastomeric article of claim 9 wherein the elastomeric article is a glove.

Fig. 1

Fig. 2

Fig. 3

Europäisches
Patentamt

European
Patent Office

Office européen
des brevets

# EUROPEAN SEARCH REPORT

**Application Number**

EP 25 19 7546

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/054079 A1 (NEUSER JOSEPH H [US] ET AL) 8 March 2007 (2007-03-08) * paragraphs [0078], [0093] - [0096], [0112] - [115]]; claims 1-4 * | 1-12 | INV.<br>C08J3/00<br>C08L1/00<br>C08J7/04<br>A41D19/00 |
| X | US 2007/053958 A1 (NEUSER JOSEPH H [US] ET AL) 8 March 2007 (2007-03-08) * paragraphs [0037] - [0039], [0087], [0094], [0097], [0113], [0114]; claims 1-9, 15-18 * | 1-12 | |

**TECHNICAL FIELDS SEARCHED (IPC)**

C08J
B29C
C08B
C08L
A41B
A41D

The present search report has been drawn up for all claims

| Place of search | Date of completion of the search | Examiner |
|---|---|---|
| Munich | 23 January 2026 | Schweissguth, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................................................

& : member of the same patent family, corresponding document

EPO FORM 1503 03.82 (P04C01)

## EP 4 707 321 A1

## ANNEX TO THE EUROPEAN SEARCH REPORT
## ON EUROPEAN PATENT APPLICATION NO.

EP 25 19 7546

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2026

| Patent document cited in search report | Publication date | Patent family member(s) | Publication date |
|---|---|---|---|
| US 2007054079 A1 | 08-03-2007 | NONE | |
| US 2007053958 A1 | 08-03-2007 | US 2007053958 A1 | 08-03-2007 |
| | | US 2010229281 A1 | 16-09-2010 |
| | | US 2012102620 A1 | 03-05-2012 |

EPO FORM P0459

For more details about this annex : see Official Journal of the European Patent Office, No. 12/82

15

**REFERENCES CITED IN THE DESCRIPTION**

**Patent documents cited in the description**

- US 20060062815 A1
- US 5614202 A
- US 20070053958 A1
- US 6274154 B1
- US 6423328 B2
- US 6630152 B2
- US 20080034467 A1

- US 8313833 B2
- WO 2006071308 A
- US 20220175068 A1
- US 20050132466 A1
- US 20060141186 A1
- CA 3042804 C
- US 10023718 B2